# EUROPEAN PATENT SPECIFICATION

(11) **EP 1 622 652 B1**
(45) Date of publication and mention of the grant of the patent: **25.07.2007**
(21) Application number: 04724921.4
(22) Date of filing: 31.03.2004
(51) Int. Cl.: A61L 15/36, A61K 35/74, A45D 37/00, A61F 13/15

(54) **Hygiene product comprising probiotic composition**
Hygieneprodukt enthaltend eine probiotische Zusammensetzung
Produit d'hygiène contenant une composition probiotique

(30) Priority: 13.05.2003 SE 0301390
(43) Date of publication of application: 08.02.2006
(73) Proprietor: SCA Hygiene Products AB, 405 03 Göteborg (SE)
(72) Inventor: HUSMARK, Ulrika, S-435 38 Mölnlycke (SE); GUSTAFSON, Ingrid, S-430 31 Asa (SE)
(74) Representative: Bergstrand, Mikael Gudmundsson
(86) International application number: PCT/SE2004/000491
(87) International publication number: WO 2004/101008

(56) References cited:
- WO-A1-99/17813
- WO-A1-99/45099
- WO-A1-03/038068
- WO-A1-03/053397
- WO-A2-01/13956
- WO-A2-03/039260
- US-A1- 2003 012 810
- US-A1- 2003 124 104

## Description

### Technical field

The present invention pertains to a hygiene product, such as a sanitary napkin, panty-liner, tampon, diaper, incontinence guard, hygiene tissue etc. characterized in that said hygiene product is provided with a probiotic composition containing a dispersion of a bacterial preparation in a lipid phase, wherein said bacterial preparation contains at least one lactic acid producing bacterial strain and contact sorption drying carrier(s). The invention also pertains to a process for producing such a hygiene product.

### Background of the invention

The urogenital area harbours a complex microbial ecosystem comprising more than 50 different bacterial species (Hill et al., Scand. J. Urol. Nephrol. 1984;86 (suppl.) 23-29). The dominating species for fertile women in this area are lactic acid producing bacteria belonging to the genus *Lactobacillus.* These lactic acid producing members are important for retaining a healthy microbial flora in these areas, and act as probiotic bacteria with an antagonistic effect against pathogenic microbial species. Lactic acid producing bacteria inhibit growth and colonization by other micro-organisms by occupying suitable niches for colonization, by forming biofilms and competing for available nutrients, thereby excluding colonization by harmful micro-organisms. Also, the production of hydrogen peroxidase, specific inhibiting substances, such as bacteriocines, and organic acids (including lactic acid and acetic acid) that lower the pH, inhibit colonization by other micro-organisms.

The microbial ecosystem of a healthy individual can be disturbed by the use of antibiotics, during hormonal changes, such as during pregnancy or use of contraceptives with estrogen, during menstruation, after menopause, in people suffering from diabetes etc. Also, micro-organisms may spread from the anus to the urogenital area, thereby causing infections. This results in a disturbance of the normal microbial flora and leaves the individual susceptible to microbial infections that cause vaginitis, urinary tract infections and ordinary skin infections. Micro-organisms commonly associated with these kinds of infections belong to the genera *Escherichia, Enterococcus, Psedomonas, Proteus, Klebsiella, Streptococcus, Staphylococcus, Gardnerella* and *Candida.* Women are at particular risk due to their shorter distance between the anus and the urogenital tract; specially at risk are young women, who not yet have a well-developed microflora in the urogenital area and older women, who no longer have a protective flora.

One way to reduce the problems with the kinds of infections described above is to have a good personal hygiene. However, excessive use of cleaning agents not only decrease the amount of harmful microbes, but can harm the beneficial microbial flora, again render it susceptible for pathogenic species to colonize and cause infections. Alternatively, administration of lactic acid producing bacteria to the urogenital area and the skin in order to outcompete pathogenic species and facilitate reestablishment and maintenance of a beneficial microbial flora in these areas, have been found to be a successful means to treat and prevent microbial infections.

It has been suggested that lactic acid producing bacteria can be delivered via absorbent products, such as diapers, sanitary napkin, incontinence guards, panty liners and tampons, as described in, for example, in WO92/13577, WO97/02846, WO99/17813 WO99/45099 and WO00/35502.

A major problem with providing products intended to be used for transfer of lactic acid producing bacteria, is that the bacteria have to retain viability during transport and storage of the products. A major problem with products comprising lactic acid producing bacteria is that the bacteria rapidly lose viability under moist conditions, and it is therefore important that the products are not exposed to moisture. One way to partly overcome this problem has been to supply products with freeze-dried lactic acid producing bacteria. However, if the bacteria in the products are not protected from moisture after manufacturing of the products, the air humidity will subsequently kill the bacteria and the shelf-life of such products will then be shortened. Another disadvantage with the direct application of dried lactic acid producing bacteria to a hygiene product, such as an absorbent product, is that transfer of the bacteria to the urogenital area will be low.

In order to overcome the problem with air humidity decreasing the shelf-life of products containing lactic acid producing bacteria it has been suggested to prepare dispersions of lactic acid producing bacteria and a hydrophobic substance, such as a fat or an oil. Research experiments have shown that storage in sterile vaseline oil results in a high level of viable lactobacilli cells after 8 months of storage (Arkadéva et al., N A. Nauchnye Doklady Vysshei Shkoly. Biologicheskie Nauki, 1983, 2:101-104). However, Stoianova et al. (Mikrobiologiia, 2000, 69:98-104), found that immersion in mineral oil was not effective to preserve viability of lactic acid producing bacteria. US 4,518,696 describes liquid suspensions of Lactobacilli in sunflower oil for oral administration to animals. However, none of the above references are concerned with the problems associated with retaining a high viability of lactic acid producing bacteria on hygiene products to be used to administer lactic acid producing bacteria to the urogenital area of a subject.

There are additional examples of the combination lactic acid producing bacteria and an oil, although these do not describe the effect of the oil on the survival of the lactic acid producing bacteria. WO01/13956 describes the use of pharmaceutical compositions comprising Emu oil, antimicrobial agents and/or *Bacillus coagulans* to be used for antimicrobial treatments. However, WO01/13956 does not describe how the problem with loss of viability during storage of the resulting products is to be solved. WO02/28446 described the use of an essentially hydrophobic carrier and freeze-dried lactic acid producing bacteria to prepare a distribution to be applied to an absorbent product. The hydrophobic carrier was mainly chosen to overcome problems with applying the bacteria to the absorbent product during manufacturing, but the carrier also protects the bacteria from air humidity.

As discussed above products comprising lactic acid producing bacteria often contain freeze-dried bacteria since a high moisture content in the product result in products with shorter shelf-life due to reduced survival. Freeze-drying, however, is an expensive and complicated way of preparing bacteria with low moisture content. RU 2104299 describes an alternative method of drying bacteria, wherein the bacteria are mixed with contact sorption drying carrier before air-drying of the product. Nothing is disclosed in RU 2104299 about reducing the water content of the bacterial preparation by transferring the bacteria to a non-aqueous phase after the drying process.

In conclusion, there is still a need to develop products for delivery of lactic acid producing bacteria to urogenital area that are convenient to use, result in efficient transfer of the bacteria to the area where they are applied and that can be stored for long time periods without loss of viability of the bacterial cells. In addition, the manufacturing processes used to manufacture these products today are inefficient and expensive and there is a need to develop these to reduce manufacturing costs.

### Summary of the invention

The above defined problems are solved in the present invention by using contact sorption drying carrier(s) for drying lactic acid producing bacteria, thereby improving the manufacturing process of hygiene products comprising lactic acid producing bacteria. The present invention disclose a hygiene product comprising a bacterial composition comprising lactic acid producing bacteria and contact sorption drying carrier(s) dispersed in a lipid phase. By using this approach a hygiene product is obtained wherein the lactic acid producing bacteria are protected from moisture and thereby has a prolonged shelf-life.

### Definitions

By "contact sorption drying carriers" are meant substances that have the ability to take up moisture from the ambient environment. The contact sorption drying carriers as used in the present invention, take up moisture from bacterial cells and bacterial microenvironments. Examples of contact sorption drying carriers suitable for the present invention include oligo- and polysaccharides and inorganic agents. Further details of contact sorption drying carrier are given below.

By "hygiene product" is meant hygiene products such as sanitary napkins, incontinence guards, tampons, panty-liners, diapers, incontinence guards, hygiene tissues etc.

By "probiotic composition" or "bacterial composition" is meant a composition comprising probiotic bacteria, i.e. bacteria that have the ability to re-establish the natural microbial flora of the host. The probiotic composition according to the present invention further comprises a lipid phase and contact sorption drying carrier(s).

By "dispersion" is meant a mixture of at least two phases.

By "preparation of at least one lactic acid producing bacterial strain", "bacterial preparation", "preparation of bacteria", "bacterial powder preparation" is meant a preparation comprising at least one lactic acid producing bacterial strain and a contact sorption drying carrier(s).

Preferred "lactic acid producing bacteria" for the object of the present invention includes bacteria from the genera *Lactobacillus, Lactococcus* and *Pediococcus.* Preferably the selected bacterium used is from the species *Lactococcus lactis, Lactobacillus acidophilus, Lactobacillus curvatus* or *Lactobacillus plantarum.* More pref erably the bacterial strain is selected from *Lactobacillus plantarum.* Even more preferably the lactic acid producing bacterium is *Lactobacillus plantarum* 931 (deposition No. (DSMZ): 11918). The bacteria are preferably isolated from the natural flora of a healthy person, preferably the bacteria are isolated from the skin or urogenital area.

By "lipid phase" is meant a water-insoluble organic phase with a fatty character. Lipids suitable to be used in the lipid phase of the invention include petroleum-derived lipids, synthetic lipids, and animal- and plant-derived lipids.

Examples of "additional components" include, but are not limited to, agents protecting the bacterial cells during drying of the bacteria, agents acting as nutrient for bacterial propagation, and skin caring agents. Further examples of suitable additional components are given below.

### Detailed description of the invention

The object of the present invention is to provide hygiene products, such as sanitary napkins, tampons, panty-liners, diapers, incontinence guards, hygiene tissues etc. suitable for absorbing bodily fluids and simultaneously delivering probiotic lactic acid producing bacteria to the skin, or more preferably, the urogenital area. The present invention pertains to solving the problems associated with providing products comprising lactic acid producing bacteria, such as problems with bacterial survival and costs and effectiveness of manufacturing. Prior art only disclose absorbent products comprising lactic acid producing bacteria in a lipid phase in which the bacteria have been freeze-dried. However, large scale freeze-drying of bacteria is not optimal since it is a complicated and expensive procedure.

The present inventors have found that by using alternative methods for drying the bacteria a cheaper and simpler process for producing hygiene products comprising lactic acid producing bacteria can be provided. In this alternative process a contact sorption drying carrier is used in the drying process in order to prepare a dried bacterial preparation that subsequently is mixed with a lipid phase and applied to a hygiene product.

The hydrophobic character of the lipid phase decreases the amount of air humidity, which reaches the bacterial cells dispersed in the lipid phase, thereby increasing the survival time for the bacteria in the probiotic composition. Dispersing the lactic acid producing bacteria in a lipid phase has the additional advantage that transfer of the bacteria to the skin and/or urogenital area is enhanced compared to when no lipid phase is used. When the product is used, the lipid phase softens when exposed to body heat and the probiotic composition is transferred to the skin. When the bacteria come in contact with moisture after delivery to the skin, they are reactivated, start to grow and perform their probiotic action.

Contact sorption drying carriers suitable for the present invention includes oligo- and polysaccharides, such as starch, maltodextrin and beta-glucane and inorganic agents, such as silicon dioxide (SiO₂). The amount of drying contact sorption carrier used is preferably between ca 10-50 % by weight when added to a bacterial suspension before drying of the bacterial preparation. A too low amount of drying contact sorption carrier would result in too long drying times and a too high amount would make the resulting bacterial powder produced after drying hard to disperse in the lipid phase.

The water activity in the bacterial preparation comprising lactic acid producing bacteria and contact sorption drying carrier is preferably 0.30 or below, more preferably 0.25 or below, most preferably 0.20 or below. The amount of bacterial preparation in the lipid phase is preferably between 1-50% by weight, more preferably 5-25% by weight, most preferably 10-20%.

The bacterial preparation comprising lactic acid producing bacteria and contact sorption drying carrier is preferably a fine powder.

Lactic acid producing bacteria are chosen for the present invention due to their positive effect in preventing and treating microbial infection in the urogenital area and on the skin. The bacteria are preferably isolated from a healthy person, preferably from the skin or urogenital area of a healthy person. Preferred "lactic acid producing bacteria" for the object of the present invention includes bacteria from the genera *Lactobacillus, Lactococcus* and *Pediococcus.* Preferably the selected bacteria are from the species *Lactococcus lactis, Lactobacillus acidophilus, Lactobacillus curvatus* or *Lactobacillus plantarum.* More preferably the selected bacterium is a *Lactobacillus plantarum* strain. Even more preferably the lactic acid producing bacterium is *Lactobacillus plantarum* 931 (deposition No. (DSMZ): 11918). The lactic acid producing bacteria can be provided alone or in mixtures containing at least two bacterial strains.

The lipid phase used in the present invention can be composed of a single lipid or a mixture of two or more lipids. Below a selection of lipids suitable for the present invention is presented.

**Table 1**

| Name | Producer | Melting range | Main ingredients |
|---|---|---|---|
| Caremelt 107 | Cognis¹⁾ | 25-58°C | Triglyceride, paraffin, monoglyceride |
| Caremelt 3 | Cognis | 30-47°C | Triglyceride, paraffin, silicone wax, liquid triglyceride |
| Caremelt 58 | Cognis | 30-49 °C | Triglycerides, polymer wax, stearyl alcohol, silicone wax |
| Vaseline | AC Hud AB²⁾ | 5-45 °C | Petrolatum |
| Beeswax | Apoteket³⁾ | | Cera Flava |
| Akosoft 36 | Karlshamn⁴⁾ | 34-3 8 °C | Vegetable fat, hard fat |
| Lipex BC | Karlshamn | 35°C | Hydrogenated Vegetable oil |
| AMS-C30 | DOW-corning⁵⁾ | 70 °C | Silicone wax |

| | | | |
|---|---|---|---|
| ¹⁾Henkel KgaA, Dusseldorf, Germany ²⁾Aco Hud AB, Stockholm, Sweden ³⁾ Apoteket AB, Produktion och Laboratorier, Gothenburg, Sweden ⁴⁾Karlshamns AB, Karlshamn, Sweden ⁵⁾Seneffc, Belgium | | | |

According to the present invention the probiotic composition is applied on a hygiene product. For this purpose it is important that the lipid phase has a melting behaviour that allows the lipid phase to support bacterial survival on the hygiene product and not disturb the absorptive power of the hygiene product. A lipid phase with too low melting point tends to spread over the product and thereby reduce the absorptive power of the product. Also, a lipid phase with a low melting point tend not to enclose the bacteria to a high enough extent when the probiotic composition is spread on the hygiene product and thereby leave a too large portion of the bacteria unprotected from air humidity, thereby reducing bacterial survival on the hygiene product. On the other hand, by choosing a lipid phase that has a melting behaviour as specified by the present invention, the lipid phase enclose the bacteria to a high enough extent. Thereby less of the bacterial population is exposed to atmospheric humidity, which result in a higher survival of the bacteria during storage of the hygiene product.

There is also an upper temperature limit for the melting behaviour of the lipid phase. This limit is in part governed by the fact that for mixing the bacterial preparation with the lipid phase, the lipid phase has to be soft enough in order to obtain a homogenous mixture. A lipid phase with a too high melting point has to be brought to temperatures that are too high for the bacteria to withstand while being mixed with the lipid phase and therefore a too large portion of the bacteria would die during preparation of the probiotic composition. Also, a lipid phase with too high melting point is not suitable for the present invention since it does not soften to a high enough extent when in contact with the skin and therefore delivery of the bacteria to the skin is impaired.

The consistency of the probiotic composition is also influenced by the bacterial preparation, which provide texture and consistency of the probiotic composition. By varying the ratio of amount of bacterial preparation to amount of lipid phase it is possible to achieve a bacterial composition with a suitable consistency for the present invention.

Therefore, preferably, the lipid phase according to the present invention is in major part solid at 30°C, becomes softer between 30°C and 40°C, but is not fully melted at 50°C, preferably not fully melted at 60°C, most preferably not fully melted at 70°C. However, the lipid phase is still exhibiting soft properties from 30°C. This melting behaviour can be achieved by using a single lipid or by mixing different lipids with different melting behaviours in order to achieve the desired melting behaviour of the lipid phase. In order for the lipid phase not to interfere with bacterial survival due to a too high water content, the water content of the lipid phase preferably is 1% by weight or less, more preferably 0.1% by weight or less. The water vapour transmission rate of the lipid phase, measured according to ASTME 398-83 at 37.8°C (100°F) and 90% relative humidity (RH), is 10 g/m²/24 h or less, more preferably 5 g/m²/24 h or less, most preferably 2 g/m²/24 h or less.

Additional components can also be a part of the probiotic composition of the present invention or applied to a hygiene product of the present invention. Examples of such additional components include, but are not limited to, agents protecting the bacterial cells during drying of the bacteria, such as sugars (e.g. maltose, glucose, sucrose, trehalose, fructose), proteins (e.g. skim milk, albumin), amino acids (e.g. sodium glutamate), polyols (e.g. xylitol), mannitol and sorbitol, pH-regulating agents (e.g. lactic acid) and antioxidants (e.g. sodium ascorbate). Additional components also include nutrients that enhance bacterial propagation once the bacteria are activated by moisture after they are delivered to the skin or urogenital area. Examples of nutrients suitable for the present invention are sugars (such as maltose, glucose, sucrose, trehalose, fructose), polysaccharides (such as starch), vitamins (such as vitamin B and E) and proteins (such as skim milk). Suitable additional components also include skin caring substances e.g. lipid soluble skin caring substances, such as vitamin A and E, skin caring oils, such as chamomile oils (Bisabolol), eucalyptus oil, lavender oil and phytosterols.

In the process for producing the hygiene product comprising a probiotic composition of the present invention, in short, a water suspension comprising at least one lactic acid producing bacterial strain (with or without additional agents protecting the bacterial cells during drying of the bacteria) is mixed with the dry contact sorption drying carrier. Immediately after addition of the contact sorption drying carrier, drying is spontaneously initiated. However to fulfil the drying, a drying step is carried out. After the drying step is carried out the dry bacterial preparation is dispersed in a lipid phase, which subsequently is added to the hygiene product.

In order to achieve a high survival rate during the drying step, the growth conditions for the bacterial cells have to be optimized in order to have bacteria with a fitness as high as possible during the drying step. The growth conditions, harvest procedures and the optional use of additional agents protecting the cells during the drying step, all affect the survival rate of the bacterial cells during drying and the subsequent storage of the probiotic composition. These conditions have to be optimized for each bacterial strain. However, such an optimization is within the skills of a person skilled in the art. The concentration of bacterial cells in the bacterial water suspension to which the contact sorption drying carrier is added is 10⁶-10¹⁵ CFU (colony forming units)/ml, preferably 10¹⁰-10¹³ CFU/ml.

The drying step carried out after addition of contact sorption drying carrier to the bacterial water suspension can be performed by e.g. convective drying methods, contact drying methods or by using electromagnetic radiation. Examples of convective drying methods suitable for the present invention include spray drying, spray granulation and fluidized bed drying. The common feature for convective drying methods is that warm and dry gas flushes around the product and enters into a heat and mass transfer with the product. Convective methods transfer required heat and/or dryness by convection to the wet product. During contact drying, the wet product is stationary in touch with a warm surface or constantly brought into new contact with the warm surface by stirring or revolving. Drying by electromagnetic radiation (infrared or microwave radiation) involves using a belt dryer or a stationary support and submitting the wet product to electromagnetic radiation energy, which is being absorbed, by the wet product. The absorbed energy serves to warm up the product whereby the moisture in the wet product is evaporated. Drying times using electromagnetic radiation often result in very short drying times. Before the drying step additional components, such as nutrients and protecting agents, can be added to the bacterial cells. Examples of such additional components are given above.

A probiotic composition is thereafter prepared wherein the dry bacterial preparation, comprising lactic acid producing bacteria and contact sorption drying carrier, is dispersed in a lipid phase. During this step further additional components, such as nutrients for bacterial propagation and skin caring substances can be added to the probiotic composition.

The probiotic composition is finally added to the hygiene product. Preferably, the probiotic composition is applied in a string(s) or patch(es), since covering a too large part of the hygiene products would result in reduced absorptive properties of the product. Preferably not more than 40% of the surface of the hygiene product is covered by the probiotic composition. More preferably not more the 20% of the surface of the hygiene product is covered by the probiotic composition.

The present invention also relates to hygiene products comprising a probiotic composition, produced by the process described above.

The probiotic composition according to the present invention is applied to a hygiene product, such as a hygiene tissue, incontinence guard, diaper, panty liner, tampon, sanitary napkin etc..

By hygiene tissue" is meant any device for wiping skin, for instance, a washcloth, patch, towelette, napkin, wetwipe, and the like. The hygiene tissue provided can be composed of a matrix comprising any natural or synthetic fiber, such as rayon, cellulose, regenerated cellulose, polyester, polyolefine fibers, textile and the like, or foam, nonwoven, felt or batting, or combinations thereof. The probiotic composition according to the present invention is applied or impregnated to the hygiene tissue matrix.

The probiotic composition of the present invention, is, as described above, particularly suitable for application to absorbent products, such as sanitary napkins, incontinence guards, panty-liners, diapers, tampons etc, since these products provide a convenient means for delivery of lactic acid bacteria to the urogenital area. The sanitary napkins, incontinence guards, panty-liners, tampons and diapers according to the invention preferably comprise of a liquid permeable casing sheet facing the user and an absorbent layer comprised of one or more layers, placed beneath or inside the liquid permeable casing sheet. The probiotic composition is placed onto the casing sheet or inside it.

Below a more detailed description of an absorbent product, such as a sanitary napkin, panty liner, diaper or incontinence guard is given. The absorbent product includes a liquid-permeable casing sheet or top sheet disposed on that side of the absorbent product which is intended to lie proximal to the wearer in use. The liquid-permeable casing sheet will conveniently consist in a somewhat soft, skin-friendly material. Different types of non-woven material are examples of suitable liquid-permeable materials. Other casing sheet materials that can be used are perforated plastic films, net, knitted, crocheted or woven textiles, and combinations and laminates of the aforesaid types of material.

The absorbent product also includes a liquid-impermeable casing sheet or backing sheet, disposed on that side of the napkin distal from the wearer in use. The liquid-impermeable casing sheet is conventionally comprised of thin plastic film. Alternatively, there may be used a liquid-permeable material that has been rendered impermeable to liquid in some way or another. For instance, the liquid-permeable material may be coated with a glue that is impermeable to liquid, and the liquid-permeable layer laminated with a liquid-impermeable material, or hot-calendering a material that was initially liquid-permeable, such as to melt down the surface of the material and therewith obtain a liquid-impermeable layer. Alternatively, there may be used other textiles comprised of hydrophobic fibers and so impervious as to enable them to be used as a liquid barrier layer. The liquid-impermeable casing sheet may beneficially be vapor permeable.

The two casing sheets form a joining edge that projects outwardly around the napkin contour line, and are mutually joined at this edge. The sheets may be joined together by means of any appropriate conventional technique, such as gluing, welding or sewing.

The absorption core sandwiched between the casing sheets shall constitute the layer capable of receiving and storing essentially all liquid discharged by the wearer. The absorption core may, for instance, be produced from cellulose pulp. This pulp may exist in rolls, bales or sheets that are dry-defibred and converted in a fluffed state to a pulp mat, sometimes with an admixture of superabsorbents, which are polymers capable of absorbing several times their own weight of water or body liquid (fluid). Examples of other usable materials are different types of foamed materials known, for instance, from SE 9903070-2, natural fibers, such as cotton fibers, peat, or the like. It is, of course, also possible to use absorbent synthetic fibers, or mixtures of natural fibers and synthetic fibers. Patent Application SE 9903070-2 describes a compressed foam material of regenerated cellulose, e.g. viscose. Such foam material will preferably have a density of 0.1 to 2.0 g/cm³. The absorbent material may also contain other components, such as foam-stabilizing means, liquid-dispersing means, or a binder, such as thermoplastic fibers, for instance, which have been heat-treated to hold short fibers and particles together so as to form a coherent unit.

A fastener means in the form of an elongate rectangular region of self-adhesive is provided on the surface of the liquid-impermeable casing sheet that lies distal from the wearer in use. The fastener means extends over the major part of the liquid-impermeable casing sheet. The invention is not restricted to the extension of the fastener means, and said means may have the form of elongate stripes, transverse regions, dots, circles, or other patterns and configurations. Neither is the invention restricted to the use of solely adhesive fastener means, since friction fasteners may be used and other types of mechanical fasteners, such as press studs, clips, girdles, pants or the like may be used when found suitable to do so. When an adhesive fastener means is used this is commonly protected, by a protective layer, from adhering to other surfaces prior use, which would destroy the fastener means.

One way to place the probiotic composition is exemplified, wherein the probiotic composition is placed in strings.

In a similar manner to what is described above, a tampon comprising the probiotic composition can be prepared. For instance, the probiotic composition according to the present invention can be arranged onto the casing sheet in strings.

The skilled person could easily use the above exemplary descriptions of the hygiene products described above which comprise a probiotic composition according to the present invention, to manufacture a tampon, sanitary napkin or any other hygiene product comprising a probiotic composition according to the invention. Therefore, alternative designs of a sanitary napkin, incontinence guard, panty-liner, diaper, tampon, hygiene tissue etc are also included in the present invention.

In one preferred embodiment of the present invention, a lipid phase (which comprises or does not comprise lactic acid producing bacteria) which melts at a higher temperature is placed underneath the probiotic composition. The lower lipid phase then functions as a protecting layer that inhibit the probiotic composition to be spread over the hygiene product when the probiotic composition melts. Thereby, the risk of inhibition of absorption by the lipid phase is even more decreased, since the lower lipid phase does not melt, or melts to a lower extent, than the upper phase, when in contact with body heat or during storage at elevated temperatures.

In addition to enhancing bacterial survival, the use of a lipid phase in which the lactic acid producing bacteria are dispersed also enhances transfer rates of the probiotic composition to the skin and urogenital area. This can be an effect of the lipid having more "adhesive" properties than, for example, water, thereby resulting in a higher amount of bacteria actually being transferred to the skin. Also, the lipid has the effect of enhancing bacterial survival once the bacteria are delivered to the skin, presumably because the lipid creates a micromilieu, that is beneficial for retaining bacterial viability and that enhances growth of the bacteria once added to the skin.

The positive effect of performing the present invention lies in advantages associated with the manufacturing of products comprising lactic acid producing bacteria (since the present invention solves many of the problems associated with the production of such products). The use of contact sorption drying carriers may improve the survival rate of the bacterial cells during the drying step, probably by stabilizing the cell membrane. In addition, the use of contact sorption drying carriers can allow a fine powder of dried bacteria to be achieved directly (a fine powder is necessary if the dried bacteria are to be mixed with a lipid phase). If the dried bacteria achieved is not in the form of a fine powder, which is the case after the commonly used freeze-drying, a further step of grinding or sifting is necessary to produce the fine powder. This adds stress to the bacteria, and, during the extra time required to perform the grinding or sifting the bacteria may take up moisture from the environment, which might adversely affect bacterial survival. In comparison, by using a contact sorption carrier according to the present invention a fine powder can be achieved directly, thereby avoiding the grinding/sifting step. The use of a contact sorption drying carriers can also speed up the drying process and aid in reaching a lower water activity, which is an advantage in terms of bacterial survival and economy. Also, the use of contact sorption drying carriers can provide a more economical means for drying bacterial cells, since sublimation of water (as in freeze-drying) is more energy consuming than evaporation, which is used in the present invention. In addition the investment costs for a freeze-drying plant are high. It can also be difficult to apply freeze-drying in a continuous process, and such a process can be advantageous when the production of large amounts of dry bacteria is necessary.

Therefore, in conclusion, by producing the hygiene products comprising probiotic composition according to the present invention, several advantages in terms of economy, simplicity in manufacturing and bacterial survival during manufacturing and during subsequent storage, can be achieved.

The present invention will now be described by the use of illustrative examples that are not intended to be limiting for the present invention.

### Example 1: Procedure for drying with SiO₂ as contact sorption drying carrier and convective drying:

An aqueous suspension of *L. plantarum* 931 (deposition No. (DSMZ): 11918) in 10% Na-glutamate and 10% glucose was used as a starting material. The cell concentration was 6.5x10¹¹ cfu/ml. 80 ml of the suspension was mixed with 160 ml of Si0₂ (Aerosil 200, Degussa Norden AB, Malmö, Sweden). Drying was performed in box with circulated dry air (33°C, 0.8% relative humidity). The bacterial powder preparation was dry after approx. 6-8 hours. The final amount of bacterial preparation was 27 g with 2.4x10¹¹ cfu/g, which corresponds to a calculated survival rate of 12%. The water activity of the powder was 0.034 measured with an equipment from Aqualab, model 3TE (Decagon Devices Inc., Pullman, WA, USA).

### Example 2: Comparison of convective drying and IR-drying with and without different contact sorption drying carriers

The bacteria (*L. plantarum* 931 (deposition No. (DSMZ): 11918)) were suspended in deionized water or in an aqueous solution of 10% trehalose. The contact sorption drying carriers tested were aerosil 200 (Degussa Norden AB, Malmö, Sweden), native potato starch and β-glucane. The cell concentration was 10¹³-10¹⁴ cfu/ml. 2.5g aerosil was added to 20 ml of cell suspension, 30g β-glucane was added to 25 ml cell suspension and 27 g potato-starch was added to 25 ml cell suspension, in order to achieve a "porridge" with a consistency convenient to handle. The final drying of the bacterial preparation to a powder was performed either via convective drying (40°C, for maximum 3 hours) or with fast drying with IR (infrared light, 40-58 °C for 5-7 minutes).

The results of the experiment are presented in Table 2. A very satisfactory survival rate of the bacteria was achieved. The addition of trehalose did not affect the survival rate of the bacteria. Addition of sugars during drying of bacterial cells is commonly considered necessary. However, in the drying process of the present invention, this was not necessary. Both drying methods, convective drying and IR drying, respectively, gave similar results in terms of survival rates. The use of aerosil or potato starch resulted in the best powder structure (i.e. a more fine powder). All the used contact sorption drying carriers resulted in high survival rates.

**Table 2**

| Sugar | Drying carrier | Drying method | Final A_{w} | CFU/g powder | % survival |
|---|---|---|---|---|---|
| Trehalose | Aerosil | IR | 0.19 | 2.2E13 | 3.9 |
| - | Aerosil | IR | 0.26 | 2.7E13 | 4.7 |
| Trehalose | β-glucan | Convective | 0.19 | 5.2E12 | 20.8 |
| - | β-glucan | Convective | 0.20 | 5.4E12 | 54.2 |
| Trehalose | β-glucan | IR | 0.26 | 1.0E12 | 3.9 |
| - | β-glucan | IR | 0.24 | 2.5E12 | 27.2 |
| Trehalose | Pot.starch | Convective | 0.16 | 1.0E14 | 83 |
| - | Pot.starch | Convective | 0.17 | 5.0E14 | 100 |
| Trehalose | Pot.starch | IR | 0.22 | 4.0E14 | 100 |
| - | Pot.starch | IR | 0.20 | 1.0E13 | 52 |

### Example 3: Comparison of drying efficacy with and without contact sorption drying carrier

A water suspension of *L. plantarum* 931 (deposition No. (DSMZ): 11918) with a concentration of 5.7x10¹¹ cfu/ml was mixed with the contact sorption drying carriers, aerosil (Degussa) and potato starch, respectively, and dried in a convective drying chamber (34°C and 1% relative humidity) for 24 hours. The water activity was determined after the drying (i.e. after 24 hours).

The results of Example 3 are presented in Table 3. As can be seen in Table 3, the water activity was one order of magnitude higher when no contact sorption drying agent was used. The water activity according to the present invention is preferably 0.30 or below and such low water activity levels could not be obtained without the use of a contact sorption drying carrier according to the present invention.

**Table 3**

| Sugar | Drying carrier | A_{w} in powder |
|---|---|---|
| Trehalose 10% | None | 0.45 |
| Trehalose 10% | Aerosil | 0.038 |
| Trehalose 10% | Pot.starch | 0.027 |
| Sorbitol 10% | None | 0.258 |
| Sorbitol 10% | Aerosil | 0.041 |
| Sorbitol 10% | Pot.starch | 0.022 |

### Example 4: Convective spray-drying

A water suspension of *L. plantarum* 931 (deposition No. (DSMZ): 11918) was mixed with 10% maltodextrine and spray-dried at two different air temperatures. The feed-flow was 5 ml/minute and the airflow was 8001/minute. The resulting powder was very fine (grain size approximately 5-10 µm). The survival rates of this experiment are presented in Table 4 below.

**Table 4**

| Contact sorption drying agent | Temp in/ Temp out | A_{w} | Cfu/g powder | % survival |
|---|---|---|---|---|
| Maltodextrine | 180/90 | 0.12 | 2.5E10 | 17 |
| Maltodextrine | 150/75 | 0.16 | 9.6E10 | 61 |

### Example 5: Survival in a bacterial composition during long term storage.

A bacterial powder preparation was produced with the process described in Example 1. The bacterial powder was dispersed in different lipid phases (beeswax, Caremelt 58, Caremelt 107, or vaseline) directly after production by melting the lipid phase and dispersing the powder in the lipid phase (1 g powder to 9 g lipid phase). The resulting bacterial composition was poured into open glass vials and allowed to solidify. The vials were very loosely covered with a aluminum-foil and placed in a standard climate chamber (23°C, 50% relative humidity). At certain times (during a time period of 8 months) the number of surviving cells was measured. For sampling, 1g of bacterial composition was scraped from the surface, placed in 9 ml of NaCl, stomached for 1 minute and the number of CFU was measured with spread-plate technique on MRS-agar.

Very high survival rates are obtained.

### Example 6: Transfer of a bacterial composition of the present invention from a panty-liner surface to skin.

The bacterial powder preparation was produced with the process described in example 1. The powder was directly after the production dispersed in different melted waxes (1g powder to 9g wax). With a pilot scale printer the produced bacterial composition was printed to the surface layer of panty-liners. The printed pattern was dots (ca 3 mm in diameter, distance ca 3 mm)

The transfer test was performed with specimens, a circle of 2.5 cm in diameter, punched out from the products. 10 µl of NaCl was added to the product with a pipette, and the specimen subsequently mounted, with constant pressure, (elastic tape, and elastic bandage), on to the forearm of volunteers. After 2 hours the product was removed and the number of lactobacilli on the skin measured. A sterile stainless-steel cylinder (2.6 cm in diameter, height 2 cm) was held tight to the skin at the site that had been covered with the specimen, and 1 ml of phosphate buffer (0.1M, pH 7.2) was poured into the cylinder. With a smooth glass-stick the skin was gently "kneaded" for 1 minute. Afterwards, the buffer was collected with a pipette and the CFU measured with pour plate technique and on MRS-agar. The amount of indigenous lactic acid producing bacteria in this place of the body is very low compared to the amount of bacteria transferred and therefore all the counted bacteria were taken as transferred bacteria.

The percentage of bacterial cells in the bacterial composition was calculated as follows:
X=number of CFU/specimen before transfer test
Y=number of transferred CFU
Percentage transfer=Y/X

As a control, a specimen wherein the bacteria were not mixed with a lipid phase before placement on the panty-liner was used. Instead, as a control specimen, a panty-liner to which a bacterial suspension (without contact sorption drying carrier) had been applied whereafter the panty-liner with the applied bacteria had been dried was used.

**Table 5**

| Lipid phase | Percentage transfer |
|---|---|
| Carmelt 3 | 22.8 |
| Caremelt 58 | 47.9 |
| Vaseline | 89.6 |
| Beeswax | 27.0 |
| Control | 2.13 |

As can be seen in Table 5 the amount of bacteria transferred to the skin from the panty-liner provided with the probiotic composition of the present invention results in a very high transfer rates of bacterial cells from the product to the skin.

## Claims

1. A hygiene product, such as a sanitary napkin, panty-liner, tampon, diaper, incontinence guard, hygiene tissue, etc. **characterized in that** said hygiene product is provided with a probiotic composition containing a dispersion of a bacterial preparation in a lipid phase, said bacterial preparation containing at least one lactic acid producing bacterial strain and contact sorption drying carrier(s) chosen from the group of oligo- and polysaccharides, such as starch, maltodextrin and beta-glucane, and inorganic agents such as silicon dioxide (SiO₂).

2. A hygiene product according to claim 1 wherein the lactic acid producing bacterium is isolatable from the skin or urigenital area of a healthy person.

3. A hygiene product according to claims 1-2 wherein the lactic acid producing bacterial strain is selected from the genera *Pediococcus, Lactococcus, Lactobacillus* or mixtures thereof.

4. A hygiene product according to claim 3 wherein the lactic acid producing bacterial strain is selected from at least *Lactobacillus plantarum.*

5. A hygiene product according to claim 4 wherein the lactic acid producing bacterial strain is at least *Lactobacillus plantarum* 931 (deposition No. (DSMZ): 11918).

6. A hygiene product according to claim 1-5 further comprising additional components chosen from agents protecting bacterial cells during drying, agents acting as nutrient for bacterial propagation, and skin caring substances.

7. A hygiene product according to claim 1-6, wherein the water activity of the bacterial preparation is 0.30 or below, more preferably 0.25 or below, most preferably 0.20 or below.

8. A hygiene product according to claims 1 - 7, wherein the water content of the lipid phase preferably is 1 % by weight or less, more preferably 0.1 % by weight or less.

9. A hygiene product according to claims 1- 8, wherein the water vapour transmission rate of the lipid phase, measured according to ASTME 398-83 at 37.8 °C (100 °F) and 90% relative humidity (RH), is 10 g/m²/24 h or less, more preferably 5 g/m²/24 h or less, most preferably 2 g/m²/24 h or less.

10. A process for producing a hygiene product according to anyone of claims 1-9, such as a sanitary napkin, panty-liner, tampon, diaper, incontinence guard, hygiene tissue stc., comprising a dispersion of a preparation of at least one lactic acid producing bacterial strain and contact sorption drying carrier(s) chosen from the group of oligo- and polysaccharides, such as starch, maltodextrin and beta-glucane, and inorganic agents such as silicon dioxide (SiO₂) in a lipid phase comprising the steps of:
a) mixing at least one lactic acid producing bacterial strain with contact sorption drying carrier(s);
b) drying the bacterial preparation comprising said at least one lactic acid producing bacterial strain and said contact sortption drying carrier(s);
c) dispersing said bacterial preparation in said lipid phase, thereby producing a probiotic composition; and
d) applying said resulting probiotic composition to said hygiene product.

## Patentansprüche

1. Hygieneprodukt, wie eine (Damen-)Binde, Slipeinlage, Tampon, Windel, Inkontinenzschutz, Reinigungstuch usw., **dadurch gekennzeichnet, daß** das Hygieneprodukt mit einer probiotischen Zusammensetzung versehen ist, die eine Dispersion einer bakteriellen Zubereitung in einer Lipidphase umfaßt, wobei die bakterielle Zubereitung zumindest einen Milchsäure produzierenden Bakterienstamm und einen oder mehrere durch Kontaktsorption trocknende(n) Träger, der/die aus der Gruppe von Oligo- und Polysacchariden, wie Stärke, Maltodextrin und beta-Glucan, und anorganischen Mitteln, wie Siliziumdioxid (SiO₂), ausgewählt ist/sind, enthält.

2. Hygieneprodukt gemäß Anspruch 1, worin das Milchsäure produzierende Bakterium aus der Haut oder dem urogenitalen Bereich einer gesunden Person isolierbar ist.

3. Hygieneprodukt gemäß den Ansprüchen 1 bis 2, worin der Milchsäure produzierende Bakterienstamm aus den Gattungen *Pediococcus, Lactococcus, Lactobacillus* oder Mischungen hiervon ausgewählt ist.

4. Hygieneprodukt gemäß Anspruch 3, worin der Milchsäure produzierende Bakterienstamm zumindest aus *Lactobacillus plantarum* ausgewählt ist.

5. Hygieneprodukt gemäß Anspruch 4, worin der Milchsäure produzierende Bakterienstamm zumindest *Lactobacillus plantarum* 931 (Hinterlegungsnr. (DSMZ): 11918) ist.

6. Hygieneprodukt gemäß Anspruch 1 bis 5, ferner umfassend zusätzliche Komponenten, ausgewählt aus Mitteln, die bakterielle Zellen während des Trocknens schützen, Mitteln, die als Nährstoffe für das bakterielle Wachstum dienen, und Hautpflegesubstanzen.

7. Hygieneprodukt gemäß Anspruch 1 bis 6, worin die Wasseraktivität der bakteriellen Zubereitung 0,30 oder weniger, stärker bevorzugt 0,25 oder weniger, am stärksten bevorzugt 0,20 oder weniger, beträgt.

8. Hygieneprodukt gemäß Ansprüchen 1 bis 7, worin der Wassergehalt der Lipidphase vorzugsweise 1 Gew.% oder weniger, stärker bevorzugt 0,1 Gew.% oder weniger, beträgt.

9. Hygieneprodukt gemäß Ansprüchen 1 bis 8, worin die Wasserdampf-Transmissionsrate der Lipidphase, gemessen gemäß ASTME 393-83 bei 37,8°C (100°F) und 90 % relativer Luftfeuchtigkeit (RH), 10 g/m²/24 h oder weniger, stärker bevorzugt 5 g/m²/24 h oder weniger, am stärksten bevorzugt 2 g/m²/24 h oder weniger, beträgt.

10. Verfahren zur Herstellung eines Hygieneprodukts gemäß irgendeinem der Ansprüche 1 bis 9, wie einer (Damen-)Binde, einer Slipeinlage, einem Tampon, einer Windel, einem Inkontinenzschutz, einem Reinigungstuch usw., umfassend eine Dispersion einer Zubereitung von zumindest einem Milchsäure produzierenden Bakterienstamm und einem oder mehreren durch Kontaktsorption trocknenden Träger(n), ausgewählt aus der Gruppe von Oligo- und Polysacchariden, wie Stärke, Maltodextrin und beta-Glucan, und anorganischen Mitteln, wie Siliziumdioxid (SiO₂) in einer Lipidphase, welches die Schritte umfaßt:
a) Mischen von zumindest einem Milchsäure produzierenden Bakterienstamm mit einem oder mehreren durch Kontaktsorption trocknenden Träger(n);
b) Trocknen der bakteriellen Zubereitung, die den zumindest einen Milchsäure produzierenden Bakterienstamm und den einen oder mehrere durch Kontaktsorption trocknende(n) Träger umfaßt;
c) Dispergieren der bakteriellen Zubereitung in der Lipidphase, wodurch eine probiotische Zusammensetzung hergestellt wird; und
d) Aufbringen der resultierenden probiotischen Zusammensetzung auf das Hygieneprodukt.

## Revendications

1. Produit d'hygiène, comme une serviette hygiénique, un protège-slip, un tampon, une couche, une protection anti-incontinence, un mouchoir en papier, etc., **caractérisé en ce que** ce produit d'hygiène est muni d'une composition probiotique contenant une préparation bactérienne à l'état de dispersion dans une phase lipidique, laquelle préparation bactérienne contient au moins une souche bactérienne productrice d'acide lactique et un ou des support(s) desséchant(s) agissant par contact et sorption, choisi(s) dans l'ensemble formé par les oligosaccharides et les polysaccharides, comme l'amidon, la maltodextrine et le bêta-glucane, et les agents inorganiques comme le dioxyde de silicium (SiO₂)

2. Produit d'hygiène conforme à la revendication 1, dans lequel la bactérie productrice d'acide lactique peut être isolée sur la peau ou dans la zone urogénitale d'une personne en bonne santé.

3. Produit d'hygiène conforme à la revendication 1 ou 2, dans lequel la souche bactérienne productrice d'acide lactique est choisie parmi celles des genres *Pediococcus, Lactococcus, Lactobacillus* et leurs associations.

4. Produit d'hygiène conforme à la revendication 3, dans lequel la souche bactérienne productrice d'acide lactique est choisie parmi, au moins, *Lactobacillus plantarum.*

5. Produit d'hygiène conforme à la revendication 4, dans lequel la souche bactérienne productrice d'acide lactique est au moins *Lactobacillus plantarum* 931 (n° de dépôt DSMZ : 11918).

6. Produit d'hygiène conforme à l'une des revendications 1 à 5, qui contient en outre des composants supplémentaires choisis parmi des agents qui protègent les cellules bactériennes pendant un séchage, des agents qui ont un rôle de nutriments pour la croissance bactérienne, et des substances de soin pour la peau.

7. Produit d'hygiène conforme à l'une des revendications 1 à 6, dans lequel l'activité de l'eau dans la préparation bactérienne vaut au plus 0,30, mieux encore au plus 0,25 et surtout au plus 0,20.

8. Produit d'hygiène conforme à l'une des revendications 1 à 7, dans lequel la teneur en eau de la phase lipidique vaut de préférence au plus 1 % en poids, et mieux encore au plus 0,1 % en poids.

9. Produit d'hygiène conforme à l'une des revendications 1 à 8, dans lequel la vitesse de transfert de la vapeur d'eau à travers la phase lipidique, mesurée selon la norme ASTM E 398-83 à 37,8 °C (100 °F) et sous 90 % d'humidité relative (HR), vaut au plus 10 g/m² par 24 heures, mieux encore au plus 5 g/m² par 24 heures et surtout au plus 2 g/m² par 24 heures.

10. Procédé de fabrication d'un produit d'hygiène conforme à l'une des revendications 1 à 9, comme une serviette hygiénique, un protège-slip, un tampon, une couche, une protection anti-incontinence, un mouchoir en papier, etc., comprenant une dispersion, dans une phase lipidique, d'une préparation d'au moins une souche bactérienne productrice d'acide lactique et d'un ou de support(s) desséchant(s) agissant par contact et sorption, choisi(s) dans l'ensemble formé par les oligosaccharides et les polysaccharides, comme l'amidon, la malto-dextrine et le bêta-glucane, et les agents inorganiques comme le dioxyde de silicium (Si0₂), lequel procédé comporte les étapes suivantes :
a) mélanger au moins une souche bactérienne productrice d'acide lactique avec un ou des support(s) desséchant(s) agissant par contact et sorption ;
b) faire sécher la préparation bactérienne contenant ladite souche bactérienne productrice d'acide lactique au nombre d'au moins une et ledit ou lesdits support(s) desséchant(s) agissant par contact et sorption ;
c) disperser ladite préparation bactérienne dans ladite phase lipidique, ce qui donne une composition probiotique ;
d) et appliquer la composition probiotique ainsi obtenue sur ledit produit d'hygiène.
